# FASCICULE DE BREVET EUROPEEN

(11) **EP 2 776 116 B1**
(45) Date de publication et mention de la délivrance du brevet: **27.05.2020**
(21) Numéro de dépôt: 12783214.5
(22) Date de dépôt: 07.11.2012
(51) Int. Cl.: A61M 39/10, F16L 33/22, A61M 39/00

(54) **ENSEMBLE DE CONNEXION**
VERBINDUNGSANORDNUNG
CONNECTION ASSEMBLY

(30) Priorité: 08.11.2011 FR 1160164
(43) Date de publication de la demande: 17.09.2014
(73) Titulaire: Vygon, 95440 Ecouen (FR)
(72) Inventeur: COUSSEGAL, Jean-Louis, F-95250 Beauchamp (FR); MENAND, Simon, F-75011 Paris (FR); GUILON, Samuel, F-95500 Gonesse (FR); CARREZ, Jean-Luc, F-95440 Ecouen (FR)
(74) Mandataire: Regimbeau
(86) Numéro de dépôt international: PCT/EP2012/072018
(87) Numéro de publication internationale: WO 2013/068393

(56) Documents cités:
- GB-A- 2 379 253
- GB-A- 2 451 891
- US-A1- 2003 100 858
- US-B1- 6 440 126

## Description

L'invention concerne de manière générale des ensembles de connecteurs pour assemblage conique et à filetage de verrouillage, utilisables pour réaliser des connexions de transmission de fluide dans le domaine de l'appareillage médical.

Plus précisément, l'invention concerne un ensemble de connecteurs mâle et femelle, adapté notamment pour des connexions neuraxiales. L'ensemble de connecteurs n'est cependant pas limité à cette application particulière, et peut également être utilisé dans d'autres dispositif médicaux, tels que des dispositifs de perfusion, des cathéters intraveineux ou artériels, des lignes de nutrition entérales ou encore tout accessoire lié à l'anesthésie péridurale ou à la rachianesthésie.

Des normes ont été établies pour l'ensemble de ces raccords, leur imposant notamment certaines dimensions et formes.

La norme NF EN 20 594 par exemple concerne des ensembles de connecteurs conçus pour réaliser des assemblages coniques avec une conicité imposée à 6% (Luer) et définit notamment les diamètres d'entrée minimal et maximal des raccords.

Dans leur version à verrouillage (« Luer Lock»), les connecteurs femelles normalisés présentent une tête qui détermine un conduit d'entrée du connecteur et sur la face extérieure de laquelle est formée un filetage, le diamètre extérieur de cette tête correspondant ainsi au diamètre des filets au sommet, tandis que les connecteurs mâles normalisés présentent un embout en saillie qui détermine un conduit d'entrée du raccord mâle et qui est entouré d'une collerette fixe ou mobile, laquelle détermine autour de l'embout une gorge pour recevoir la tête du connecteur femelle correspondant et sur la face intérieure de laquelle est formé le filetage du connecteur, de sorte que le diamètre au sommet des filets du connecteur mâle délimite le diamètre de passage de la gorge du connecteur.

Le fait que ces normes s'appliquent indifféremment aux connecteurs pour perfusion veineuse ou aux raccords pour nutrition entérale est une cause potentielle d'accident.

Il peut arriver en effet qu'un conteneur contenant un produit anesthésiant soit utilisé pour alimenter une sonde pour nutrition entérale par exemple, ou au contraire qu'une seringue de nutriment ou tout autre produit autre qu'un anesthésiant soit utilisée accidentellement pour alimenter une ligne de perfusion ou un cathéter veineux ou artériel.

Par exemple, le document US 6 440 126 propose un ensemble de connecteurs mâle et femelle comprenant chacun une collerette. Les collerettes comprennent des filets qui s'étendent en regard, depuis une surface interne de la collerette mâle et depuis une surface externe du connecteur femelle, afin de permettre leur connexion. Néanmoins, ces connecteurs sont très similaires à ceux utilisés dans d'autres domaines et ne permettent donc pas à l'opérateur de se rendre compte facilement d'une erreur au moment de la connexion.

Différentes mesures ont été préconisées pour éviter ces connexions indésirables.

Par exemple, on a pensé à distinguer les raccords par des codes de couleurs, ce qui s'avère en pratique être une précaution insuffisante.

En ce qui concerne les seringues, on a proposé de les munir d'une extrémité Luer femelle au lieu de l'extrémité Luer mâle habituelle.

Le document GB 2 383 828 quant à lui a proposé un kit de connexion comprenant :
- une seringue, comportant, de manière permanente, un premier convertisseur présentant un embout mâle « différent »,
- un second convertisseur, comprenant un embout femelle « différent » adapté pour se connecter avec l'embout mâle « différent », et un embout femelle standard, et
- une aiguille comprenant un embout mâle standard.

Le kit peut en outre comprendre un convertisseur supplémentaire, présentant le même embout mâle « différent » que le premier connecteur, ainsi qu'un embout femelle standard, afin de permettre la connexion à des composants supplémentaires munis eux-mêmes de connecteurs standards, tels qu'un filtre.

Par embout mâle « différent », il faut comprendre embout différent d'un embout standard. Par exemple, il est possible d'utiliser un embout mâle de diamètre inférieur à celui de l'embout mâle standard. De la sorte, les embouts ne peuvent alors pas être verrouillés ensemble.

Néanmoins, seule l'insertion de l'embout femelle différent dans un embout mâle standard est empêchée, alors que l'insertion de l'embout mâle différent dans l'embout femelle standard est dans ce cas facilitée, ce qui va à l'encontre du but recherché. En variante, le document propose l'utilisation d'un anneau de contrôle permettant d'empêcher le verrouillage de l'embout mâle différent à un embout femelle standard grâce à des éléments de filetage adaptés pour ne coopérer qu'avec un épaulement externe de l'embout femelle différent. Cependant, rien n'empêche l'embout du raccord mâle de pénétrer dans le conduit d'entrée du raccord femelle.

On a donc proposé dans le document FR 2 863 162 un nouveau système de raccords, adapté de préférence aux lignes de nutrition entérale, comprenant des raccords mâle et femelle pour assemblage conique et à filetages de verrouillage présentant des diamètres d'entrée et au sommet des filets choisis par rapport aux diamètres correspondants des raccords normalisés en sorte que l'assemblage d'un raccord mâle ou femelle de ce système soit empêché parce que la pénétration de l'embout du raccord mâle dans le conduit d'entrée du raccord femelle est impossible, ou parce que cette pénétration est arrêtée par butée de la tête du raccord femelle contre une collerette du raccord mâle.

Il a également été proposé, dans le document US 2003/0100858, un ensemble de connexion réutilisable formé d'un connecteur mâle et d'un connecteur femelle comprenant chacun une collerette. Le connecteur femelle présente en outre un embout apte à pénétrer dans la collerette du connecteur mâle. Par ailleurs, la collerette du connecteur mâle et la collerette du connecteur femelle présentent des moyens filetés complémentaires, et les moyens filetés de la collerette du connecteur mâle s'étendent radialement depuis une surface externe de ladite collerette, tandis que les moyens filetés de la collerette du connecteur femelle s'étendent radialement depuis une surface interne de ladite collerette. Néanmoins, le connecteur mâle ne comprend pas d'embout adapté pour pénétrer l'embout du connecteur femelle. Il s'agit simplement d'un conduit, destiné à venir en regard de l'embout femelle pour permettre une connexion fluidique.

Le document GB 2 451 891 quant à lui propose un ensemble de connexion comprenant un connecteur mâle comprenant un embout mâle, et un connecteur femelle comprenant un embout femelle. Chacun des connecteurs est muni d'une collerette, munis de moyens de fixation à baïonnettes. Un tel changement de mode de fixation des connecteurs n'est cependant pas rassurant pour un opérateur, qui est plus habitué à visser les connecteurs entre eux.

Il a également été proposé dans le document US 6 440 126 un système de cryocathéter comprenant un premier raccord, un second raccord et un cathéter. L'extrémité distale du premier raccord peut être couplée à l'extrémité proximale du second raccord pour les placer en communication fluidique, et l'extrémité distale du second raccord peut être couplée à l'extrémité proximale du cathéter pour les placer en communication fluidique.

Enfin, il a été proposé dans le document GB 2 379 253 un raccord pour un système d'administration de fluide médical destiné à l'administration de liquides à un patient, qui comprend des éléments mâle et femelle, l'élément femelle possédant une douille conique se rétrécissant vers l'intérieur destinée à recevoir un ergot tubulaire complémentaire de l'élément mâle, ainsi qu'une encoche d'échappement de liquide destinée à laisser échapper du liquide apporté par un élément mâle défectueux ou incorrectement raccordé.

La présente invention a notamment pour objet d'améliorer encore les ensembles de connecteurs médicaux de l'art antérieur de manière à mieux empêcher encore les connexions indésirables accidentelles, tout en proposant un ensemble de connecteurs mâle et femelle présentant une bonne étanchéité et assurant une connexion sécurisée évitant tout desserrage accidentel ou non souhaité.

Pour cela, l'invention propose un ensemble de connexion conforme à la revendication 1 annexée

Certains aspects préférés mais non limitatifs de l'ensemble de connexion selon l'invention sont les suivants :
- une surface externe de l'embout mâle est convergente en direction d'une extrémité distale dudit embout mâle, tandis qu'une surface interne de l'embout femelle est divergente en direction d'une extrémité distale dudit embout femelle ;
- une surface interne de la collerette d'embout mâle est divergente en direction d'une extrémité distale de la collerette d'embout mâle, tandis qu'une surface externe de l'embout femelle est convergente en direction d'une extrémité distale dudit embout femelle de manière à créer un effet de coin lorsque le connecteur mâle et le connecteur femelle sont connectés ;
- une conicité de la surface interne de la collerette d'embout mâle est sensiblement égale à une conicité de la surface externe de l'embout femelle ;
- une extrémité distale de l'embout mâle affleure une extrémité distale de la collerette d'embout mâle ;
- le connecteur mâle est réalisé dans un élastomère thermoplastique ;
- les collerettes sont formées d'un seul tenant avec les embouts respectifs ; et
- les extrémités proximales des collerettes se trouvent sensiblement au droit des extrémités proximales des embouts respectifs.

Selon d'autres aspects, l'invention propose un conteneur équipé d'un connecteur femelle d'un ensemble de connexion conforme à l'invention, le conteneur pouvant être constitué par une seringue, ainsi qu'un cathéter équipé d'un connecteur femelle d'un ensemble de connexion conforme à l'invention ou encore un prolongateur équipé d'un connecteur d'un ensemble de connexion conforme à l'invention.

D'autres caractéristiques, buts et avantages de la présente invention apparaîtront mieux à la lecture de la description détaillée qui va suivre, faite en référence aux dessins annexés donnés à titre d'exemples non limitatifs et sur lesquels :
La figure 1a représente une vue en coupe d'un exemple de réalisation d'un connecteur mâle conforme à l'invention ;
La figure 1b représente une vue en perspective de la figure 1a ;
La figure 2a représente une vue en coupe d'un exemple de réalisation d'un connecteur femelle conforme à l'invention
La figure 2b une vue en perspective de la figure 2a ;

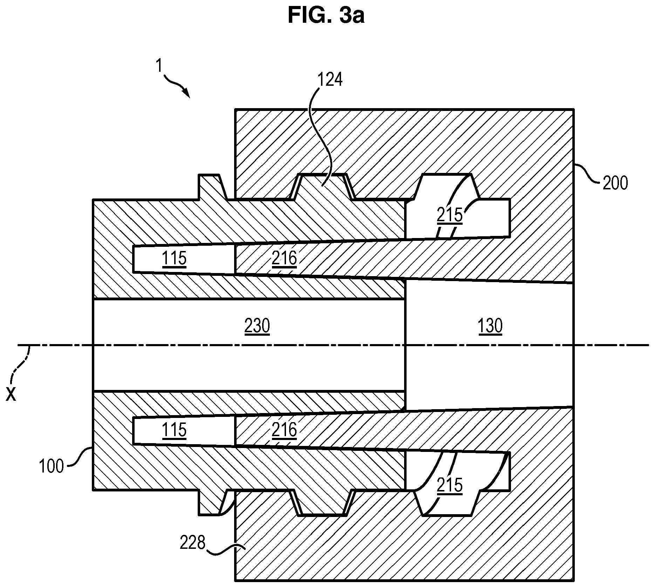

La figure 3a représente une vue en coupe d'un ensemble formé par le connecteur mâle des figures 1a et 1b et le connecteur femelle des figures 2a et 2b ; et

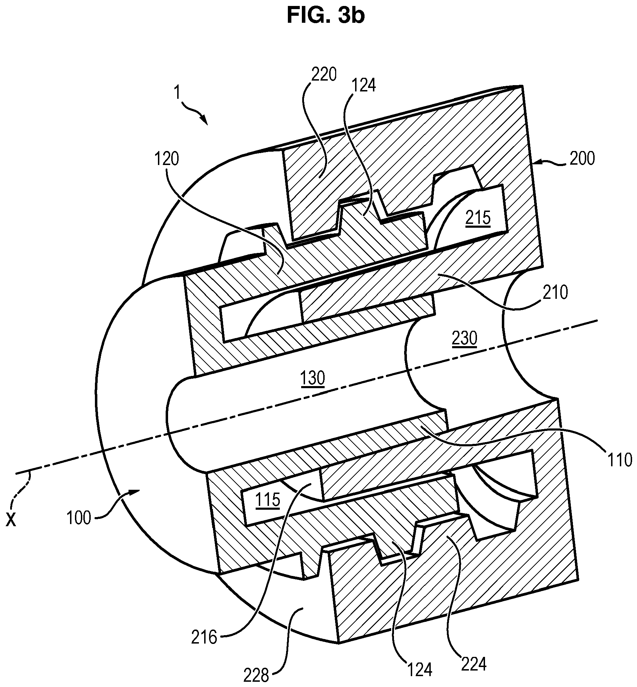

La figure 3b une vue en perspective de la figure 3a.

En référence aux figures, nous allons à présent décrire un exemple de réalisation d'un ensemble de connexion 1 conforme à l'invention, comprenant un connecteur mâle 100 et un connecteur femelle 200.

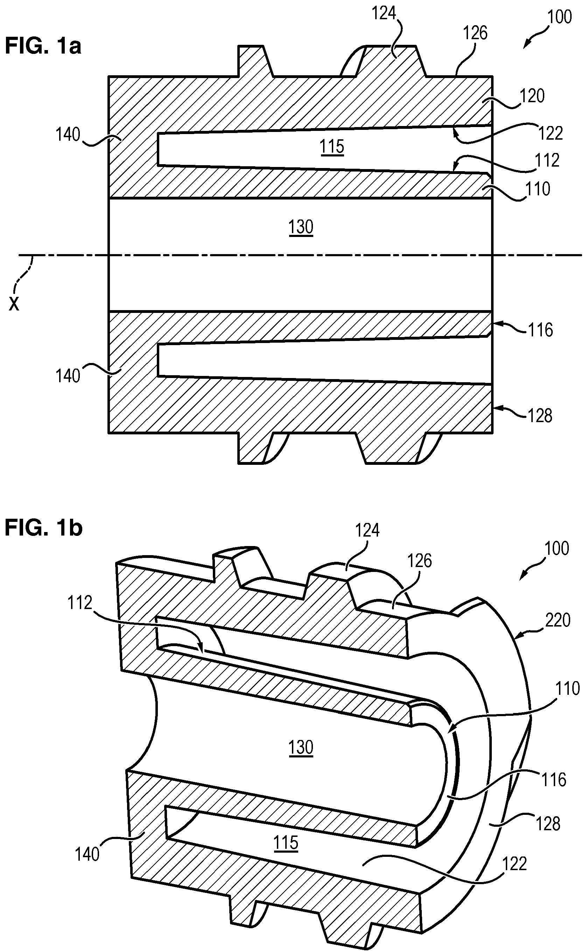

Comme visible sur les figures 1a et 1b, un connecteur mâle 100 conforme à l'invention comprend un embout 110 mâle conique, autour duquel est formée une collerette d'embout mâle 120.

Par connecteur mâle, on comprendra un connecteur comprenant un embout mâle adapté pour pénétrer dans un embout complémentaire femelle. Un connecteur n'est donc pas mâle par le simple fait qu'il puisse ou non pénétrer dans un connecteur complémentaire.

L'embout mâle 110 est creux et définit un passage 130 pour un fluide, par exemple un anesthésiant. Par ailleurs, la collerette 120 entoure l'embout mâle 110 de manière à définir, entre une face interne 122 de la collerette 120 et une face externe 112 de l'embout mâle 110 une gorge 115 globalement cylindrique, par exemple de forme globalement conique.

La collerette 120 comprend en outre un ou plusieurs filets 124 s'étendant radialement en saillie depuis sa surface externe 126.

L'embout mâle 110 et sa collerette 120 présentent tous deux une extrémité distale 116 et 128 respectivement qui s'étendent dans un même plan normal à un axe de révolution X du connecteur mâle 100. En variante, l'extrémité distale 116 de l'embout mâle 110 est en saillie par rapport à l'extrémité distale 228 de la collerette 220, ou inversement.

A l'extrémité proximale, l'embout 110 et sa collerette 120 sont réunies par une partie de liaison 140 en forme de couronne. Par ailleurs, la collerette 120 peut être formée d'un seul tenant avec l'embout 110.

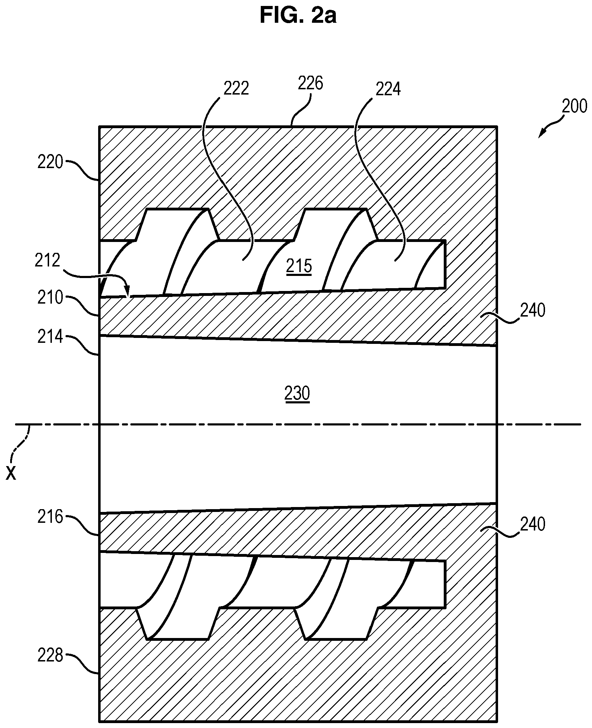

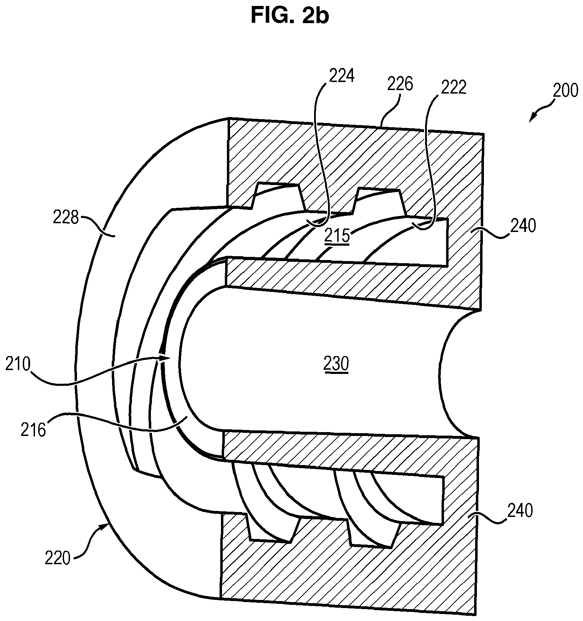

En se référant à présent aux figures 2a et 2b, un connecteur femelle 200 conforme à l'invention comprend un embout 210 femelle conique, autour duquel est formée une collerette d'embout femelle 220.

L'embout femelle 210 est creux et définit un passage 230 pour recevoir l'embout mâle 110 et pour un fluide, par exemple un anesthésiant. Par ailleurs, la collerette 220 entoure l'embout femelle 210 de manière à définir, entre une face interne 222 de la collerette 220 et une face externe 212 de l'embout femelle 210 une gorge 215 globalement conique, adaptée pour recevoir la collerette 120 du connecteur mâle 100.

La collerette femelle 220 comprend en outre un ou plusieurs filets en creux 224 s'étendant radialement depuis sa surface interne 226, adaptés pour coopérer avec le ou les filets 124 de la collerette 120.

L'embout femelle 210 et la collerette femelle 220 présentent en outre tous deux une extrémité distale 216 et 228 respectivement qui s'étendent dans un même plan normal à un axe de révolution X du connecteur femelle 200. En variante, l'extrémité distale 216 de l'embout femelle est en saillie par rapport à l'extrémité distale 228 de la collerette femelle 220, ou inversement.

A l'extrémité proximale, l'embout 210 et sa collerette 220 sont réunies par une partie de liaison 240 en forme de couronne. Par ailleurs, la collerette 220 peut être formée d'un seul tenant avec l'embout 210.

Les dimensions des embouts mâle 110 et femelle 210 et de leurs collerettes respectives 120 et 220 sont adaptées pour que, lors de la connexion du connecteur mâle 100 et du connecteur femelle 200 :
- l'embout mâle 110 pénètre dans l'embout femelle 210,
- l'embout femelle pénètre dans la gorge 115 définie par la collerette 120 et l'embout mâle 110, et
- la collerette 120 pénètre dans la gorge 215 définie par la collerette 220 et l'embout femelle 210.

A cet effet, le diamètre interne de l'embout femelle 210 peut par exemple correspondre sensiblement au diamètre externe de l'embout mâle 110, tandis que son diamètre externe peut correspondre sensiblement au diamètre interne de la collerette 120. Par ailleurs, le diamètre au sommet du ou des filets 124 de la collerette 120 peut correspondre sensiblement au diamètre au fond des filets 224 de la collerette 220, tandis que le diamètre au fonds des filet(s) de la collerette 120 peut correspondre sensiblement au diamètre au sommet du ou des filets 224.

Par exemple, pour le connecteur mâle 100, le diamètre interne de l'embout mâle 110 peut être de 2.25 mm, pour un diamètre externe au niveau de l'entrée conique de l'embout mâle 110 de l'ordre de 3.25 mm et une pente de l'ordre de 1° 25' 56 s, tandis que le diamètre interne au niveau de l'entrée conique de la collerette 120 est de l'ordre de 5.10 mm pour une pente de 1° 25' 56 s.

Pour le connecteur femelle, le diamètre interne au niveau de l'entrée conique de l'embout femelle 210 peut être de 3.45 mm pour un diamètre externe conique de l'ordre de 4.9 mm et des pentes in de l'ordre de 1° 25' 56 s.

Par ailleurs, le diamètre au fond des filets de 124 de la collerette 120 est sensiblement égal au diamètre au sommet des filets 224 de la collerette 220, par exemple de l'ordre de 7 à 7.3 mm, tandis que le diamètre au sommet des filets 124 de la collerette 120 est sensiblement égal à celui au fond des filets 224 de la collerette 220, par exemple de l'ordre de 8.2 à 8.5 mm. Enfin, les filets 124 et 224 peuvent être hélicoïdaux, selon un pas de l'ordre de 6 mm.

On obtient alors des connecteurs 100, 200 faciles à assembler, assurant une bonne communication fluidique et présentant un effet de coin lors de leur assemblage.

Par ailleurs, la présence d'un ou de plusieurs filets sur la face externe 124 de la collerette mâle 120 constitue un moyen efficace pour distinguer le connecteur mâle 120 des connecteurs normalisés et habituellement utilisés par des opérateurs médicaux. En effet, comme indiqué dans le préambule de la description, les connecteurs mâles Luer Lock normalisés présentent un filetage sur la face interne de leur collerette, et sont extérieurement lisses. Un opérateur peut ainsi facilement se rendre compte visuellement qu'il commet éventuellement une erreur dans le choix du connecteur.

Par ailleurs, une telle structure des connecteurs 100 et 200 permet d'empêcher la connexion d'un connecteur mâle (respectivement femelle) normalisé à un connecteur femelle 200 (respectivement mâle 100) conforme à l'invention, et vice-versa, puisque leur verrouillage ne sera pas possible en raison de la position des filetages sur les connecteurs 100 et 200 conformes à l'invention. On remarquera par ailleurs qu'il n'est pas davantage possible d'assembler un connecteur mâle standard (respectivement femelle) avec un connecteur mâle (respectivement femelle) conforme à l'invention, et ce malgré la position des filetages, qui, dans ce cas, se trouveraient pourtant en regard, dans la mesure où les dimensions des embouts interdiront formellement l'insertion de l'un dans l'autre.

Selon une forme de réalisation, la surface externe 112 de l'embout mâle 110 est convergente en direction de son extrémité distale 116, tandis que la surface interne 214 de l'embout femelle 210 est divergente en direction de son extrémité distale 226, la conicité de la surface externe 112 correspondant sensiblement à la conicité de la surface interne 214, de sorte que l'on obtient un double assemblage conique. Ainsi, lors de l'assemblage des connecteurs mâle 100 et femelle 200, l'insertion de l'embout mâle 10 dans l'embout femelle 210 est plus étanche que si les embouts 110 et 20 étaient de forme tubulaire.

La conicité des embouts mâle 110 et femelle 210 peut par exemple être de 6% (Luer).

En variante ou en complément, la surface interne 122 de la collerette 120 peut être divergente en direction de son extrémité distale 128, tandis que la surface externe 212 de l'embout femelle 210 peut être convergente en direction de son extrémité distale 216, la conicité de la surface interne 122 correspondant sensiblement à la conicité de la surface externe 212.

La combinaison de ces deux formes de réalisation permet d'améliorer encore davantage l'étanchéité de l'ensemble de connexion. En effet, l'ensemble de connexion 1 présente alors une double étanchéisation (entre l'embout mâle 110 et l'embout femelle 210 d'une part, et entre l'embout femelle 120 et la collerette 220 par l'effet de coin d'autre part) ainsi qu'un hyperstatisme lors de l'assemblage des connecteurs mâle 100 et femelle 200.

Plus particulièrement, lors de l'assemblage, l'insertion du connecteur mâle 100 dans le connecteur femelle 200 tend à écarter les parois de la collerette mâle, les parois de l'embout mâle 110 restant sensiblement fixes en raison de leur faible déformation sous des efforts radiaux en direction de l'axe X du connecteur mâle 100. De par cette déformation, la collerette 120 applique alors une pression radiale sur la collerette 220 et comprime le ou les filets 124 du connecteur mâle contre le ou les filets 224 du connecteur femelle 200. Par conséquent, le vissage de la collerette 120 dans la collerette 220 crée une pression radiale supplémentaire ayant pour effet de réduire les risques éventuels de dévissage intempestif des connecteurs.

Afin d'améliorer l'effort de pression appliqué par la collerette 120 sur la collerette 220 au moment de l'assemblage des connecteurs 100 et 200, la collerette 120 peut par exemple être réalisée dans un matériau plus souple que le reste du connecteur 100, tel qu'un élastomère thermoplastique. En variante, l'ensemble du connecteur mâle peut être réalisé dans un tel matériau.

Par ailleurs, chaque connecteur peut comporter, au niveau de son extrémité opposée à son embout, un logement adapté pour recevoir un tube (variante non illustrée sur les figures).

Bien entendu, la présente invention n'est nullement limitée aux formes de réalisation décrites ci-dessus et représentées sur les dessins, mais l'homme du métier saura y apporter de nombreuses variantes et modifications.

## Revendications

1. Ensemble de connexion (1) formé d'un connecteur mâle (100) et d'un connecteur femelle (200) pour réaliser une transmission de fluide dans le domaine de l'appareillage médical, notamment pour une connexion neuraxiale, dans lequel :
- le connecteur mâle (100) comprend un embout mâle conique creux (110) autour duquel est formée une collerette d'embout mâle (120),
- le connecteur femelle comprend un embout femelle creux (210) autour duquel est formée une collerette femelle (220),
- un diamètre externe de l'embout mâle (110) étant sensiblement égal à un diamètre interne de l'embout femelle (210) de sorte que l'embout mâle (110) est apte à pénétrer dans l'embout femelle (210), et
- un diamètre interne de la collerette d'embout mâle (120) étant sensiblement égal à un diamètre externe de l'embout femelle (210), de sorte que l'embout femelle (210) est apte à pénétrer dans la collerette d'embout mâle (120),
la collerette d'embout mâle (120) et la collerette d'embout femelle (220) présentant des moyens filetés (124, 224) complémentaires, et en ce que les moyens filetés (124) de la collerette d'embout mâle (120) s'étendent radialement depuis une surface externe (126) de ladite collerette d'embout mâle (120), tandis que les moyens filetés (224) de la collerette d'embout femelle (220) s'étendent radialement depuis une surface interne (226) de ladite collerette d'embout femelle (220),
l'ensemble de connexion étant **caractérisé en ce que** l'embout femelle est conique et **en ce qu'**une surface interne (122) de la collerette d'embout mâle (120) est divergente en direction d'une extrémité distale (128) de la collerette d'embout mâle (120), tandis qu'une surface externe (212) de l'embout femelle (210) est convergente en direction d'une extrémité distale (228) dudit embout femelle (210) de manière à créer un effet de coin lorsque le connecteur mâle (100) et le connecteur femelle (200) sont connectés, une conicité de la surface interne (122) de la collerette d'embout mâle (120) étant sensiblement égale à une conicité de la surface externe (212) de l'embout femelle (210)..

2. Ensemble de connexion (1) selon la revendication 1, dans lequel une surface externe (112) de l'embout mâle (110) est convergente en direction d'une extrémité distale (116) dudit embout mâle (110), tandis qu'une surface interne (214) de l'embout femelle (210) est divergente en direction d'une extrémité distale (228) dudit embout femelle (210).

3. Ensemble de connexion (1) selon la revendication 2, dans lequel une conicité de la surface externe (112) de l'embout mâle (110) est sensiblement égale à une conicité de la surface interne (214) de l'embout femelle (210).

4. Ensemble de connexion (1) selon l'une des revendications 1 à 3, dans lequel une extrémité distale (116) de l'embout mâle (110) affleure une extrémité distale (116) de la collerette d'embout mâle (120).

5. Ensemble de connexion (1) selon l'une des revendications 1 à 4, dans lequel le connecteur mâle (100) est réalisé dans un élastomère thermoplastique.

6. Ensemble de connexion (1) selon l'une des revendications 1 à 5, dans lequel les collerettes (120, 220) sont formées d'un seul tenant avec les embouts (110, 210) respectifs.

7. Ensemble de connexion (1) selon l'une des revendications 1 à 6, dans lequel les extrémités proximales des collerettes (120, 220) se trouvent sensiblement au droit des extrémités proximales des embouts (110, 210) respectifs.

8. Conteneur équipé d'un connecteur femelle (200) d'un ensemble de connexion (1) selon l'une des revendications 1 à 7.

9. Conteneur selon la revendication 8, constitué par une seringue.

10. Cathéter équipé d'un connecteur femelle (200) d'un ensemble de connexion (1) selon l'une des revendications 1 à 7.

11. Prolongateur équipé d'un connecteur (100, 200) d'un ensemble de connexion (1) selon l'une des revendications 1 à 7.

## Patentansprüche

1. Verbindungsanordnung (1), gebildet von einem männlichen Verbinder (100) und von einem weiblichen Verbinder (200), um eine Fluidübertragung auf dem Gebiet der medizinischen Gerätschaft durchzuführen, insbesondere für eine neuraxiale Verbindung, wobei:
- der männliche Verbinder (100) eine hohle konische männliche Spitze (110) umfasst, um die ein männlicher Spitze-Kragen (120) ausgebildet ist,
- der weibliche Verbinder eine hohle weibliche Spitze (210) umfasst, um die ein weiblicher Kragen (220) ausgebildet ist,
- ein Außendurchmesser der männlichen Spitze (110) etwa gleich einem Innendurchmesser der weiblichen Spitze (210) ist, so dass die männliche Spitze (110) imstande ist, in die weibliche Spitze (210) einzudringen, und
- ein Innendurchmesser des männlichen Spitze-Kragens (120) etwa gleich einem Außendurchmesser der weiblichen Spitze (210) ist, so dass die weiblichen Spitze (210) imstande ist, in den männlichen Spitze-Kragen (120) einzudringen,
wobei der männliche Spitze-Kragen (120) und der weibliche Spitze-Kragen (220) komplementäre Gewindemittel (124, 224) aufweisen und dass sich die Gewindemittel (124) des männlichen Spitze-Kragens (120) ab einer Außenfläche (126) des männlichen Spitze-Kragens (120) radial erstrecken, wohingegen sich die Gewindemittel (224) des weiblichen Spitze-Kragens (220) ab einer Innenfläche (226) des weiblichen Spitze-Kragens (220) radial erstrecken,
wobei die Verbindungsanordnung **dadurch gekennzeichnet ist, dass** die weibliche Spitze konisch ist und dass eine Innenfläche (122) des männlichen Spitze-Kragens (120) in Richtung eines distalen Endes (128) des männlichen Spitze-Kragens (120) divergiert, wohingegen eine Außenfläche (212) der weiblichen Spitze (210) in Richtung eines distalen Endes (228) der weiblichen Spitze (210) konvergiert, so dass ein Keileffekt geschaffen wird, wenn der männliche Verbinder (100) und der weibliche Verbinder (200) verbunden sind, wobei eine Konizität der Innenfläche (122) des männlichen Spitze-Kragens (120) etwa gleich einer Konizität der Außenfläche (212) der weiblichen Spitze (210) ist.

2. Verbindungsanordnung (1) nach Anspruch 1, wobei eine Außenfläche (112) der männlichen Spitze (110) in Richtung eines distalen Endes (116) der männlichen Spitze (110) konvergiert, wohingegen eine Innenfläche (214) der weiblichen Spitze (210) in Richtung eines distalen Endes (228) der weiblichen Spitze (210) divergiert.

3. Verbindungsanordnung (1) nach Anspruch 2, wobei eine Konizität der Außenfläche (112) der männlichen Spitze (110) etwa gleich einer Konizität der Innenfläche (214) der weiblichen Spitze (210) ist.

4. Verbindungsanordnung (1) nach einem der Ansprüche 1 bis 3, wobei ein distales Ende (116) der männlichen Spitze (110) mit einem distalen Ende (116) des männlichen Spitze-Kragens (120) bündig ist.

5. Verbindungsanordnung (1) nach einem der Ansprüche 1 bis 4, wobei der männliche Verbinder (100) aus einem thermoplastischen Elastomer hergestellt ist.

6. Verbindungsanordnung (1) nach einem der Ansprüche 1 bis 5, wobei die Kragen (120, 220) mit den jeweiligen Spitzen (110, 210) einstückig gebildet sind.

7. Verbindungsanordnung (1) nach einem der Ansprüche 1 bis 6, wobei sich die proximalen Enden der Kragen (120, 220) etwa senkrecht zu den proximalen Enden der jeweiligen Spitzen (110, 210) befinden.

8. Behälter, ausgestattet mit einem weiblichen Verbinder (200) einer Verbindungsanordnung (1) nach einem der Ansprüche 1 bis 7.

9. Behälter nach Anspruch 8, bestehend aus einer Spritze.

10. Katheter, ausgestattet mit einem weiblichen Verbinder (200) einer Verbindungsanordnung (1) nach einem der Ansprüche 1 bis 7.

11. Verlängerung, ausgestattet mit einem Verbinder (100, 200) einer Verbindungsanordnung (1) nach einem der Ansprüche 1 bis 7.

## Claims

1. A connection unit (1) consisting of a male connector (100) and a female connector (200) for conveying fluid in the field of medical device, in particular for a neuraxial connection, wherein:
- the male connector (100) comprises a hollow conical male end-piece (110) around which a male end-piece collar (120) is formed,
- the female connector comprises a hollow female end-piece (210) around which a female collar (220) is formed,
- an outer diameter of the male end-piece (110) being substantially equal to an inner diameter of the female end-piece (210) so that the male end-piece (110) is capable of penetrating the female end-piece (210), and
- an inner diameter of the male end-piece collar (120) being substantially equal to an outer diameter of the female end-piece (210), so that the female end-piece (210) is capable of penetrating the male end-piece collar (120),
the male end-piece collar (120) and the female end-piece collar (220) having matching threaded means (124, 224), and in that the threaded means (124) of the male end-piece collar (120) extend radially from an outer surface (126) of said male end-piece collar (120), whereas the threaded means (224) of the female end-piece collar (220) extend radially from an inner surface (226) of said female end-piece collar (220),
the connection unit being **characterized in that** the female end-piece is conical and **in that** an inner surface (122) of the male end-piece collar (120) diverges towards a distal end (128) of the male end-piece collar (120), whereas an outer surface (212) of the female end-piece (210) converges towards a distal end (228) of said female end-piece (210) so as to create a wedge effect when the male connector (100) and the female connector (200) are connected, a conicity of the inner surface (122) of the male end-piece collar (120) being substantially equal to a conicity of the outer surface (212) of the female end-piece (210).

2. The connection unit (1) according to claim 1, wherein an outer surface (112) of the male end-piece (110) converges towards a distal end (116) of said male end-piece (110), whereas an inner surface (214) of the female end-piece (210) diverges towards a distal end (228) of said female end-piece (210).

3. The connection unit (1) according to claim 2, wherein a conicity of the outer surface (112) of the male end-piece (110) is substantially equal to a conicity of the inner surface (214) of the female end-piece (210).

4. The connection unit (1) according to any of claims 1 to 3, wherein a distal end (116) of the male end-piece (110) is flush with a distal end (116) of the male end-piece collar (120).

5. The connection unit (1) according to any of claims 1 to 4, wherein the male connector (100) is made of a thermoplastic elastomer.

6. The connection unit (1) according to any of claims 1 to 5, wherein the collars (120, 220) are integrally formed with the respective end-pieces (110, 210) .

7. The connection unit (1) according to any of claims 1 to 6, wherein the proximal ends of the collars (120, 220) are located substantially in line with the proximal ends of the respective end-pieces (110, 210).

8. A container equipped with a female connector (200) of a connection unit (1) according to any one of claims 1 to 7.

9. The container according to claim 8, consisting of a syringe.

10. A catheter equipped with a female connector (200) of a connection unit (1) according to any one of claims 1 to 7.

11. An extender equipped with a connector (100, 200) of a connection unit (1) according to any one of claims 1 to 7.
